# EUROPÄISCHE PATENTSCHRIFT

(11) **EP 1 485 062 B1**
(45) Veröffentlichungstag und Bekanntmachung des Hinweises auf die Patenterteilung: **23.11.2016**
(21) Anmeldenummer: 03708194.0
(22) Anmeldetag: 07.03.2003
(51) Int. Cl.: A61Q 19/00, A61Q 19/08, A61K 8/11, A61Q 1/02

(54) **WACHSBESCHICHTETE KOSMETISCHE WIRKSTOFFPARTIKEL**
WAX-COATED COSMETIC ACTIVE SUBSTANCE PARTICLES
PARTICULES DE SUBSTANCE ACTIVE COSMETIQUES ENROBEES DE CIRE

(30) Priorität: 09.03.2002 DE 10210449
(43) Veröffentlichungstag der Anmeldung: 15.12.2004
(73) Patentinhaber: Beiersdorf AG, 20245 Hamburg (DE)
(72) Erfinder: BÜRGER, Anette, 22301 Hamburg (DE); RASCHKE, Thomas, 25421 Pinneberg (DE); VOIGT, Nadine, 22455 Hamburg (DE); SCHWANKE, Frank, 20257 Hamburg (DE)
(86) Internationale Anmeldenummer: PCT/EP2003/002377
(87) Internationale Veröffentlichungsnummer: WO 2003/075881

(56) Entgegenhaltungen:
- EP-A- 0 706 821
- EP-A- 0 865 819
- WO-A-01/49407

## Beschreibung

Die vorliegende Erfindung betrifft beschichtete kosmetische und/oder dermatologische Wirkstoffpartikel, ein Verfahren zu ihrer Herstellung sowie ihre Verwendung in Kosmetika und Dermatika. Insbesondere betrifft die Erfindung wachsbeschichtete hydrophile Wirkstoffpartikel.

Zahlreiche kosmetische und/oder dermatologische Wirkstoffe sind gegenüber bestimmten Einflüssen wie Feuchtigkeit, niedrigen oder hohen pH-Werten sowie Sauerstoff nicht stabil. Es hat daher nicht an Versuchen gefehlt, derartige Wirkstoffe den Umwelteinflüssen so zu entziehen, daß dennoch bei der Anwendung eine Freisetzung der Wirkstoffe stattfindet. Ein Weg zur Erreichung dieses Ziels ist die Verkapselung von Wirkstoffen.
Zur Herstellung verkapselter kosmetischer Wirkstoffe gibt es mehrere Ansätze. Bekannt ist beispielsweise die liposomale Verkapselung von Arzneistoffen, die zu einer retardierten Wirkstofffreisetzung führen soll. Im wesentlichen handelt es sich dabei um von Phospholipiden umschlossene wirkstoffhaltige Phasen. Die Langzeitstabilität derartiger Gebilde ist gering.

Nanopartikel sind feste Partikel mit Partikelgrößen von 15 bis 300 nm. Gelegentlich werden auch größere Teilchen mit Durchmessern bis zu 1000 nm zu den Nanopartikeln gerechnet. Derartige Partikel bestehen in der Regel aus Polymeren und weisen Kavitäten auf oder bilden eine Hülle, so daß in ihrem Inneren Gastmoleküle aufhalten können, die umschlossen oder adsorbiert werden. Diese Gastmoleküle können auch Wirkstoffe sein, die bei Anwendung der Nanopartikel langsam freigesetzt werden.

Ähnlich verhalten sich feste Lipidnanopartikel, die in einer Matrix aus festen Lipiden verteilte Wirkstoffe enthalten. Die Größe der Partikel ist mit denen der Nanopatikel vergleichbar.

So beschreiben die internationale Patentanmeldung 67728 aus dem Jahr 2000 und die deutsche Offenlegungsschrift 19945203 derartige Lipidpartikel, die in ihrer Matrix Wirkstoffe enthalten. Dabei handelt es sich aber nicht um einzelne von einer Beschichtung umgebene Wirkstoffpartikel.

Die deutsche Offenlegungsschrift 4128910 beschreibt ein Verfahren zur Herstellung von Nanopartikeln durch Verdampfen einer Kolloidsuspension, die aus einer wirkstoffhaltigen wässrigen Phase und einer polymer- und lösungsmittelhaltigen Phase gebildet wurde. Über die Beschichtung von festen Partikeln mit einem nichtpolymeren Material wird hingegen nichts offenbart.

Wirbelschichten sind fluiddurchströmte Feststoffphasen, die sich ähnlich wie Flüssigkeiten verhalten. Sie haben breite technische Bedeutung erlangt: Verfahren, die auf Wirbelschichten beruhen, werden für die vielfältigsten Zwecke eingesetzt. Beispiele sind Kohlevergasung, Rösten von Kaffeebohnen, Erdnüssen oder sulfidischen Erzen, katalytisches Cracken von Kohlenwasserstoffen (FCC), Altkunststoffverwertung durch Pyrolyse, Beschichten von Werkstükken mit Kunststoffen im Wirbelsinterverfahren (Gartenstühle), Trennung von Kartoffeln von mitgeernteten Steinen, Trocknung von Getreide oder pharmazeutischen Granulaten, Verbrennung von Müll oder Kohle, Herstellung von Phthalsäureanhydrid. Um eine Wirbelschicht zu erhalten, ist es erforderlich, daß das zu fluidisierende Gut eine relativ enge Korngrößenverteilung aufweist.

Die Wirbelschicht ermöglicht es sehr effektiv, den Kontakt zwischen Feststoffen und fluiden Materialien herzustellen. Die Wirbelschichttechnologie wurde beispielsweise zur Verkapselung vor allem größerer Partikel für den Einsatz in der Nuklearindustrie eingesetzt, wobei sich aber immer mehr Verfahren zur Beschichtung keramischer sowie metallischer Puder in sehr kleinen Mikrometerbereichen etablieren. Auch findet die Wirbelschichtverkapselung Einsatz im Bereich der Maskierung von unerwünschten Geschmackseindrücken bei Pharmaka oder der Ummantelung von Arzneistoffen mit dem Zweck einer Magensaftresistenz.

Wirbelschichten ermöglichen auch die Beschichtung von festen Wirkstoffpartikeln mit einem geschmolzenen Wachs, wie es in den unten genannten Beispielen dargestellt wird.

Für den kosmetischen Einsatz eignen sich nur diejenigen Wirbelschichtverfahren, die eine Herstellung von Partikeln ermöglichen, die kleiner als 200 µm sind. Größere Partikel sind für den Anwender spürbar und weisen zumeist keine genügende kosmetische Eleganz auf.

So können mit dem von Glatt^{®} (Glatt GmbH, Binzen, Germany) in der deutschen Patentanmeldung 69217918 beschriebenen Wirbelschichtverfahren Partikelgrößen von 50 µm und kleiner erreicht werden. Bei diesem Verfahren können durch extrem hohe Sprühraten kurze Prozeßzeiten erzielt werden. Dieser sogenannte Wurster-Prozess eignet'sich als "Bottom Spray Verfahren" gegenüber den "Top Spray Verfahren" besonders gut für die Beschichtung von kleinen Partikeln.

Für den Labormaßstab mit Chargengrößen zwischen 50 g und 500 g eignen sich hier der Mini-Glatt mit Bottom- Sprayausrüstung bzw. der GPCG 1 mit Wurster Einsatz.

Mit dem Wirbelschichtcoater GPCG 5, ebenfalls mit Wurster Einsatz ausgestattet, lassen sich jeweils 5 - 10 kg Partikel im erwünschten Größenbereich unter 200 µm herstellen. Durch gezielte Prozeßführung können sogar Partikeldurchmesser von 10 µm erreicht werden.

Besonders zur Verkapselung von hydrophilen Wirkstoffen ist das in der deutschen Patentschrift 19711393 beschriebene Verfahren von Niehaus, Teipel und Krause geeignet, das mit einer unter überkritischen Bedingungen gehaltenen Wirbelschicht arbeitet. Es können hiermit Partikel im gewünschten Größenbereich mit gleichmäßigen dünnen Wandschichten von z.B. 3 µm hergestellt werden. Über auf diese Weise erhältliche kosmetische Wirkstoffpartikel wird dagenen nichts offenbart.

EP 706821 (Centre de Microencapsulation) sowie FR 2803539 (Separex) beschreiben Verkapselungstechniken von Wirkstoffen, wobei die Beschichtungsmaterialen in überkritischen Fluiden gelöst werden. Über eine vorteilhafte Anwendung eines Wirbelchschichtverfahrens wird jedoch nichts offenbart.

Ausgehend hiervon war Aufgabe der vorliegenden Erfindung, einen Weg zur Verkapselung von kosmetischen und/oder dermatologischen Wirkstoffen zu finden, welches den Nachteilen des Standes der Technik abhilft. Dies gelingt mit dem Einsatz von speziellen Wirbelschichttechnologien.

Es hat sich für den Fachmann nicht vorhersehbar herausgestellt, daß wachsbeschichtete kosmetische und/oder dermatologisch Wirkstoffpartikel erhältlich durch
(a) Behandeln der in Form einer Wirbelschicht vorgelegten unbeschichteten Wirkstoffpartikel durch Einbringen einer Lösung von Wachs in einem überkritischen Fluid, wobei das Fluid nach Eintreten in die Wirbelschicht unter überkritischen Bedingungen vorliegt und das gelöste Wachs auf den Partikeln abgeschieden wird
   oder
(b) Behandeln der in Form einer Wirbelschicht vorgelegten unbeschichteten Wirkstoffpartikel durch Einbringen von geschmolzenem Wachs in die Wirbelschicht wobei das Wachs auf den Partikeln erstarrend abgeschieden wird,
   den Nachteilen des Standes der Technik abhelfen.

Dabei ist es bevorzugt, wenn die mittlere Partikelgröße der zu beschichtenden Partikel 1 bis 200 µm, bevorzugt 5 bis 100 µm beträgt (gemessen durch statische Lichtstreuung im Mastersizer S der Firma Malvem mit einem Helium Neon Laser bei 632,8 nm in Wasser). Weiterhin bevorzugt ist es, wenn die Grenzen des Schmelzbereiches des Wachses zwischen 35 °C und 80° C liegen. Ebenso ist es bevorzugt, wenn die mittlere Schichtdicke der Beschichtung 0,001 bis 50 µm, bevorzugt 0,1-10 µm, besonders bevorzugt 0,5 bis 5 µm beträgt (gemessen durch statische Lichtstreuung im Mastersizer S der Firma Malvem mit einem Helium Neon Laser bei 632,8 nm in Wasser). Als Wirbelschichtverfahren unter Verwendung eines überkritischen Fluides wird vorteilhaft das in der Schrift DE 19711393 beschriebene verwendet, als Wirbelschichtverfahren unter Verwendung von geschmolzenem Wachs wird vorteilhaft das in der Schrift DE 69217918, insbesondere das Luftsuspensionsverfahren nach Wurster handelt, wobei das Glatt Fluid Bed Bottom Spray Coating besonders geeignet ist.

Bevorzugt sind beschichtete Partikel, bei denen der Wirkstoff eine hydrophile Substanz darstellt, insbesondere solche mit einem log P_{*Octanol*/}*_{Wasser}* kleiner 3.

Die beschriebenen Partikel sind bevorzugt erhältlich, wenn das überkritische Fluid Kohlendioxid ist, besonders bevorzugt wenn das Kohlendioxid unter einem Druck von 100 bis 200 bar und einer Temperatur von 70 bis 150°C steht.

Bevorzugt werden Wachse aus der Gruppe der natürlichen Wachse, besonders bevorzugt Bienenwachs, Carnaubauwachs, Candelillawachs, Schellackwachs, Kohlenwasserstoffe/ Paraffinwachse, besonders bevorzugt Alkane, Isoalkane, Cycloalkane,Mono-, Di - und Triglyceride aus höheren gesättigten Fettsäuren mit 10 - 30 Kohlenstoffatomen, besonders bevorzugt Glyceryltrilaureat höhere gesättigte Fettalkohole, besonders bevorzugt solche mit 14 - 30 Kohlenstoffatomen ganz besonders bevorzugt Cetostearylalkohol, synthetische Ester, besonders bevorzugt Cetylpalmitat, Polymerwachse, besonders bevorzugt Polyethylen, Polypropylen, Polyvinylether Copolymere, besonders bevorzugt solche aus Ethylen- und Vinylacetat als Beschichtung gewählt.

Bevorzugt ist es, den Wirkstoff aus der Gruppe der hydrolyse-, säure-, alkalien-, sauerstoff- oder UV-Licht-empfindliche Funktionalitäten enthaltenden Wirkstoffe, bevorzugt aus der Gruppe der Enzyme, der Peptide, der Nucleotide, der pflanzlichen Polyphenole wie beispielsweise Flavonoide oder Isoflavonoide, Kreatin, Kreatinderivate, Ascorbinsäure, Carnitin und/oder Carnitinderivate zu wählen. Die Erfindung umfasst auch kosmetische und/oder dermatologische Zubereitungen, die die beschriebenen beschichteten Partikel enthalten. Solche Zubereitungen werden bevorzugt zur Pflege der Haut, der trockenen Haut, der sensiblen Haut, der Altershaut und der UV-geschädigten Haut verwendet.

Die erfindungsgemäß hergestellten Wirkstoffpartikel stellen aus dermatologischer Sicht eine Verbesserung gegenüber Partikeln dar, die nach herkömmlichen Verkapselungsmethoden hergestellt werden, da
- durch die beschriebenen Wirbelschichtverfahren keine Verunreinigungen durch Lösungsmittel oder tensidische Substanzen in die Partikel mit eingebracht werden,
- eine definierte Wirkstoffbeladung und somit eine hohe Dosiergenauigkeit erreicht werden kann
- Wachse mit hautpflegenden Eigenschaften als Wandmaterialien eingesetzt werden können.
- eine gute Abschirmung des Wirkstoffs im Partikelkern durch die einheitliche, umgebende Hülle vor unerwünschten äußeren Einflüssen erreicht wird.

Hierbei ist es von großem Vorteil, gezielt für die Verkaspselung von kosmetischen Wirkstoffen die beschriebenen Wirbelschichtverfahren einzusetzen. Da durch Wirbelschichtcoating mit herkömmlichen Methoden entweder die Partikelgrößen zu groß ausfallen und so zu einer Beeinträchtigung der sonsorischen Wahrnehmung der Anwender von kosmetischen und/oder dermatologischen Zubereitungen führen oder das Wandmaterial nur ungleichmäßig aufgetragen wird oder es zu unerwünschten Agglomerationen der Partikel kommt, wurde bisher von Wirbelschichtverfahren für die kosmetische oder dermatologische Anwendung kein Gebrauch gemacht.

Auch ist es ein Vorteil der Erfindung, dass verkapselte Partikel mit einer einheitlichen und gleichmäßigen sowie weichen Oberfläche entstehen.

Weiter ist das Verhältnis von Wirkstoffgehalt zu Wandmaterial besonders günstig und bietet so die Möglichkeit einen höheren Gehalt an Wirkstoff zu stabilisieren, als es mit den Wachsmatrixmethoden möglich ist.

Einen besonderen Vorteil stellt außerdem die Möglichkeit dar, durch das Verfahren mit superkritischem Kohlendioxid gezielt hydrophile Wirkstoffe zu stabilisieren, die nach herkömmlichen Methoden nur sehr schwer zu verkapseln sind.

So ist es von großem Vorteil, beschichtete Partikel aus Carnitin (3-Hydroxy-4-(trimethylammonio)-buttersäurebetain), Acetylcarnitin, Creatin, hydrophile Vitamine, insbesondere Ascobinsäure, Folsäure, Liponsäure, Pyridoxin und Niacinamid und Proteasen herzustellen.

Die erfindungsgemäßen kosmetischen und/oder dermatologischen Zubereitungen finden Verwendung zur Behandlung und/oder Prophylaxe der Symptome der intrisischen und /oder extrinsischen Hautalterung, einschließlich der trockenen Altershaut, zur Reduktion von Fältchen und Falten der Haut und/oder zur Verbesserung der Elastizität der Haut sowie zur Behandlung und/oder Prophylaxe der schädlichen Auswirkungen ultravioletter Strahlung auf die Haut und zur Verbesserung des Lipidgehaltes der Haut.

Vorteilhaft können der erfindungsgemäß verwendeten Wirkstoffpartikel eingearbeitet werden in übliche kosmetische und dermatologische Zubereitungen, welche in verschiedenen Formen vorliegen können. So können sie z.B. eine wässirige oder ethanolische Lösung, eine Emulsion vom Typ Wasser-in-ÖI (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Emulsions- oder Wachsstift oder auch ein Aerosol darstellen.

Erfindungsgemäße Emulsionen im Sinne der vorliegenden Erfindung, z.B. in Form einer Creme, einer Lotion, einer kosmetischen Milch, einer Mousse-Creme aus einem Aerosolbehälter sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wässrige Systeme bzw. Tensidzubereitungen zur Reinigung der Haut und der Haare einzufügen.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Es ist dem Fachmanne natürlich bekannt, dass anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, weitere Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Herstellungsbeispiele für kosmetische Wirkstoffpartikel

I. Beschichtung von 5 kg Acetyl- L-Carnitin mit 6 kg Paraffinwachs (Tafelparaffin 52/54, Smp. 53°C) in einem Glatt^{®} Wirbelschichtcoater GPCG 5 mit Wurstereinsatz:
   Düsendurchmesser: 800 µm; Volumen: 10,5 Liter; Zulufttemperatur: 20 - 26 °C; Wirbelbetttemperatur: 32 - 35 °C; Prozeßtemperatur: 78 - 82 °C; Sprühluftdruck: 3 bar; Prozeßzeit: 74 Minuten;
      la. Beschichtung von 4 kg Acetylsalicylsäure mit 6 kg Stearylstearat (Schercemol SS) in einem Glatt^{®} Wirbelschichtcoater GPCG 5 mit Wurstereinsatz:
         Düsendurchmesser: 800 µm; Volumen: 10,5 Liter; Zulufttemperatur: 20 - 26 °C; Wirbelbetttemperatur: 32 - 35 °C; Prozeßtemperatur: 78 - 82 °C; Sprühluftdruck: 3 bar; Prozeßzeit: 74 Minuten;
      Ib. Beschichtung von 3 kg Grünteeextrakt mit 6 kg Glyceryltripalmitat in einem Glatt^{®} Wirbelschichtcoater GPCG 5 mit Wurstereinsatz:
         Düsendurchmesser: 800 µm; Volumen: 10,5 Liter; Zulufttemperatur: 20 - 26 °C; Wirbelbetttemperatur: 34 - 36 °C; Prozeßtemperatur: 80-85 °C; Sprühluftdruck: 3 bar; Prozeßzeit: 75 Minuten;
      Ic. Beschichtung von 5 kg Niacinamid mit 6 kg Glyceryltripalmitat in einem Glatt^{®} Wirbelschichtcoater GPCG 5 mit Wurstereinsatz:
         Düsendurchmesser: 800 µm; Volumen: 10,5 Liter; Zulufttemperatur: 20 - 26 °C; Wirbelbetttemperatur: 34 - 36 °C; Prozeßtemperatur: 80-85 °C; Sprühluftdruck: 3 bar; Prozeßzeit: 70 Minuten;
      Id. Beschichtung von 5 kg Kreatin mit 6 kg Stearylstearat (Schercemol SS) in einem Glatt^{®} Wirbelschichtcoater GPCG 5 mit Wurstereinsatz:
         Düsendurchmesser: 800 µm; Volumen: 10,5 Liter; Zulufttemperatur: 20 - 26 °C; Wirbelbetttemperatur: 32 - 35 °C; Prozeßtemperatur: 78 - 82 °C; Sprühluftdruck: 3 bar; Prozeßzeit: 70 Minuten;
      le. Beschichtung von 3 kg Ursolsäure mit 6 kg Glyceryltripalmitat in einem Glatt^{®} Wirbelschichtcoater GPCG 5 mit Wurstereinsatz:
         Düsendurchmesser: 800 µm; Volumen: 10,5 Liter; Zulufttemperatur: 20 - 26 °C; Wirbelbetttemperatur: 34-36 °C; Prozeßtemperatur: 80-85 °C; Sprühluftdruck: 3 bar; Prozeßzeit: 70 Minuten;
II. Beschichtung von 4,5 kg α- Liponsäure mit 5,6 kg Polyvinyl Stearyl Ether (Smp.: 45 - 55 °C) in einem Glatt^{®} Wirbelschichtcoater GPCG 5 mit Wurstereinsatz:
   Düsendurchmesser: 800 µm; Volumen: 10,5 Liter; Zulufttemperatur: 20 - 26 °C; Wirbelbetttemperatur: 32 - 35 °C; Prozeßtemperatur: 78 - 82 °C; Sprühluftdruck: 3 bar; Prozeßzeit: 68 Minuten;
   IIb. Beschichtung von 3 kg Carnosin mit 5 kg Carnaubawachs (Sheerwax® Smp.: 85 °C) in einem Glatt^{®} Wirbelschichtcoater GPCG 5 mit Wurstereinsatz:
      Düsendurchmesser: 800 µm; Volumen: 10,5 Liter; Zulufttemperatur: 20 - 26 °C; Wirbelbetttemperatur: 35 - 38 °C; Prozeßtemperatur: 80 - 85 °C; Sprühluftdruck: 3 bar;
      Prozeßzeit: 75 Minuten;
III. Beschichtung von 5 kg L-Carnitin mit 6 kg Cetylpalmitat (Smp: 48°C) in einem Glatt^{®} Wirbelschichtcoater GPCG 5 mit Wurstereinsatz:
   Düsendurchmesser: 800 µm; Volumen: 10,5 Liter; Zulufttemperatur: 20 - 26 °C; Wirbelbetttemperatur: 30 - 31 °C; Prozeßtemperatur: 72 - 76 °C; Sprühluftdruck: 3 Bar; Prozeßzeit: 75 Minuten;
   IIIa. Beschichtung von 3 kg Soja-Isoflavonoid-Extrakt mit 6 kg Stearylstearat (Schercemol SS) in einem Glatt^{®} Wirbelschichtcoater GPCG 5 mit Wurstereinsatz:
      Düsendurchmesser: 800 µm; Volumen: 10,5 Liter; Zulufttemperatur: 20 - 26 °C; Wirbelbetttemperatur: 30 - 32 °C; Prozeßtemperatur: 73 - 77 °C; Sprühluftdruck: 3 Bar; Prozeßzeit: 75 Minuten;
IV. Beschichtung von Acetyl-L-Carnitin (mittlerer Partikeldurchmesser: 11 µm) mit Kahlwachs 7050 (Olefin, Polymer; Smp: 49 - 60 °C) nach dem Verfahren zum Mikroverkapseln von Partikeln gemäß der Patentschrift DE 19711393 (nichtkontinuierliches Feststoffverfahren). Dabei wird in einem Mischungsautoklaven mit einem Volumen von 100 ml 5 g Wachs in suprafluidem Kohlendioxid bei 150 bar und 80 °C gelöst und durch eine Düse mit einem Durchmesser von 50 µm von unten in den Beschichtungstreakor eingebracht. Der zylinderförmige Beschichtungstreakor hat einen Durchmesser von 14 mm und eine Höhe von 300 mm. In diesen werden 1,4 g Acetyl-L-Carnitin vorgelegt, die im undurchströmten Zustand eine Schüttschichthöhe von 7 mm aufweisen. Das Carnitin wird bei 80 bar und 40°C mit CO₂ fluidisiert. Durch die Zugabe des im CO₂ gelösten Wachses werden die fluidisierten Wirkstoffpartikel an der Oberfläche gleichmäßig beschichtet. Man erhält Partikel, deren mittlere Schichtdikke der Kapselwand 3 µm beträgt. Der Feststoff wird durch einen Zyklon und mehrere Filter vom im Kreislauf befindlichen Kohlendioxid abgeschieden.
   IVa. Beschichtung von Folsäure (mittlerer Partikeldurchmesser: 11 µm) mit Kahlwachs 7050 (Olefin, Polymer; Smp: 49 - 60 °C) nach dem Verfahren zum Mikroverkapseln von Partikeln gemäß der Patentschrift DE 19711393 (nichtkontinuierliches Feststoffverfahren). Dabei wird in einem Mischungsautoklaven mit einem Volumen von 100 ml 5 g Wachs in suprafluidem Kohlendioxid bei 150 bar und 80 °C gelöst und durch eine Düse mit einem Durchmesser von 50 µm von unten in den Beschichtungstreakor eingebracht. Der zylinderförmige Beschichtungstreakor hat einen Durchmesser von 14 mm und eine Höhe von 300 mm. In diesen werden 1,5 g Folsäure vorgelegt, die im undurchströmten Zustand eine Schüttschichthöhe von 7 mm aufweisen. Die Folsäure wird bei 80 bar und 40°C mit CO₂ fluidisiert. Durch die Zugabe des im CO₂ gelösten Wachses werden die fluidisierten Wirkstoffpartikel an der Oberfläche gleichmäßig beschichtet. Man erhält Partikel, deren mittlere Schichtdicke der Kapselwand 3 µm beträgt. Der Feststoff wird durch einen Zyklon und mehrere Filter vom im Kreislauf befindlichen Kohlendioxid abgeschieden.
   IVb. Beschichtung von Pyridoxin (mittlerer Partikeldurchmesser: 11 µm) mit Kahlwachs 7050 (Olefin, Polymer; Smp: 49 - 60 °C) nach dem Verfahren zum Mikroverkapseln von Partikeln gemäß der Patentschrift DE 19711393 (nichtkontinuierliches Feststoffverfahren). Dabei wird in einem Mischungsautoklaven mit einem Volumen von 100 ml 5 g Wachs in suprafluidem Kohlendioxid bei 150 bar und 80 °C gelöst und durch eine Düse mit einem Durchmesser von 50 µm von unten in den Beschichtungstreakor eingebracht. Der zylinderförmige Beschichtungstreakor hat einen Durchmesser von 14 mm und eine Höhe von 300 mm. In diesen werden 1,2 g Pyridoxin vorgelegt, die im undurchströmten Zustand eine Schüttschichthöhe von 7 mm aufweisen. Das Pyridoxin wird bei 80 bar und 40°C mit CO₂ fluidisiert. Durch die Zugabe des im CO₂ gelösten Wachses werden die fluidisierten Wirkstoffpartikel an der Oberfläche gleichmäßig beschichtet. Man erhält Partikel, deren mittlere Schichtdicke der Kapselwand 3 µm beträgt. Der Feststoff wird durch einen Zyklon und mehrere Filter vom im Kreislauf befindlichen Kohlendioxid abgeschieden.
   IVc. Beschichtung von Kreatin (mittlerer Partikeldurchmesser: 11 µm) mit Kahlwachs 7050 (Olefin, Polymer; Smp: 49 - 60 °C) nach dem Verfahren zum Mikroverkapseln von Partikeln gemäß der Patentschrift DE 19711393 (nichtkontinuierliches Feststoffverfahren). Dabei wird in einem Mischungsautoklaven mit einem Volumen von 100 ml 5 g Wachs in suprafluidem Kohlendioxid bei 150 bar und 80 °C gelöst und durch eine Düse mit einem Durchmesser von 50 µm von unten in den Beschichtungstreakor eingebracht. Der zylinderförmige Beschichtungstreakor hat einen Durchmesser von 14 mm und eine Höhe von 300 mm. In diesen werden 1,1 g Kreatin vorgelegt, die im undurchströmten Zustand eine Schüttschichthöhe von 7 mm aufweisen. Das Kreatin wird bei 80 bar und 40°C mit CO₂ fluidisiert. Durch die Zugabe des im CO₂ gelösten Wachses werden die fluidisierten Wirkstoffpartikel an der Oberfläche gleichmäßig beschichtet. Man erhält Partikel, deren mittlere Schichtdicke der Kapselwand 3 µm beträgt. Der Feststoff wird durch einen Zyklon und mehrere Filter vom im Kreislauf befindlichen Kohlendioxid abgeschieden.

| **Rezepturbeispiel 1 (O/W**-**Crème)** | **Gew.-%** |
|---|---|
| Glycerylstearatcitrat | 2,00 |
| Myristylmyristat | 1,00 |
| Stearylalkohol | 2,00 |
| Cetylalkohol | 1,00 |
| Hydrierte Kokosfettglyceride | 2,00 |
| Butylenglycol Dicaprylat/Dicaprat | 1,00 |
| Ethylhexylkokosfettsäureester | 3,00 |
| Vaseline | 1,00 |
| Cyclomethicon | 3,00 |
| Dicaprylylether | 1,00 |
| TiO₂ | 1,00 |
| Ethylhexylmethoxycinnamat | 5,00 |
| Butylmethoxydibenzoylmethan | 1,00 |
| Ethylhexylsalicylat | 1,00 |
| L-Carnitin (Wachsmikrokapseln gem. Herstellungsbeispiel III) | 0,50 |
| Iminodisuccinat, Natriumsalz | 0,20 |
| Phenoxyethanol | 0,30 |
| p-Hydroxybenzoesäurealkylester (Paraben) | 0,60 |
| Diazolidinylharnstoff | 0,25 |
| Polyacrylsäure (Carbomer) | 0,05 |
| Ammoniumpolyacryloyldimethyltaurat | 0,40 |
| Glycerin | 7,00 |
| Parfüm | q.s. |
| Wasser | Ad 100,00 |

| **Rezepturbeispiel 2 (O/W-Crème)** | **Gew**.-% |
|---|---|
| Glycerylsterat, selbstemulgierend | 5,00 |
| Stearylalkohol | 1,00 |
| Sheabutter | 1,00 |
| C₁₂₋₁₅ Alkylbenzoat | 3,00 |
| Caprylsäure/Caprinsäure Triglycerid | 1,00 |
| Mineralöl | 1,00 |
| Cyclomethicon | 2,00 |
| Dicaprylylether | 3,00 |
| Ethylhexylmethoxycinnamat | 3,00 |
| Ethylhexyltriazon | 1,00 |
| Bis-Ethylhexyloxyphenol-methoxyphenyltriazin | 1,00 |
| α-Liponsäure (Wachsmikrokapseln gem. Herstellungsbeispiel II) | 0,20 |
| Citronensäure, Natriumsalz | 0,10 |
| Trinatrium EDTA | 0,10 |
| p-Hydroxybenzoesäurealkylester (Paraben) | 0,30 |
| Hexamidindiisethionat | 0,04 |
| 1,3-Dimethylol-5,5-dimethyl-hydantoin(DMDM Hydantoin) | 0,20 |
| lodopropinylbutylcarbamat | 0,10 |
| Ethanol denaturiert | 2,00 |
| Ammoniumacryloyldimethyltaurate/ Vinylpyrrolidoncopolymere | 0,50 |
| Glycerin | 6,00 |
| Butylenglycol | 1,00 |
| Wasser | Ad 100,00 |

| **Rezepturbeispiel 3 (O/W**-**Crème)** | **Gew.-%** |
|---|---|
| Glycerylsterat, selbstemulgierend | 3,00 |
| PEG-40-Stearat | 1,00 |
| Cetylalkohol | 3,00 |
| C₁₂₋₁₅ Alkylbenzoat | 2,00 |
| Caprylsäure/Caprinsäure Triglycerid | 2,00 |
| Octyldodecanol | 1,00 |
| Vaseline | 1,00 |
| Cyclomethicon | 4,00 |
| Dimethicon | 1,00 |
| Dicaprylylether | 2,00 |
| TiO₂ | 1,00 |
| Ethylhexylmethoxycinnamat | 5,00 |
| 2-Hydroxy-4-Methoxy- benzophenon | 2,00 |
| Acetyl-L-Carnitin (Wachsmikrokapseln gem. Herstellungsbeispiel I) | 0,25 |
| Milchsäure | 0,30 |
| Tocopherylacetat | 1,00 |
| Iminodisuccinat, Natriumsalz | 0,10 |
| Phenoxyethanol | 0,30 |
| p-Hydroxybenzoesäurealkylester (Paraben) | 0,20 |
| Diazolidinylharnstoff | 0,10 |
| Xanthan Gummi | 0,10 |
| Ammoniumacryloyldimethyltaurate/ Vinylpyrrolidoncopolymere | 0,30 |
| Glycerin | 7,00 |
| Wasser | Ad 100,00 |

| **Rezepturbeispiel 4 (O/W-Crème)** | **Gew.-%** |
|---|---|
| Glycerylsterat, selbstemulgierend | 1,00 |
| Stearinsäure | 4,00 |
| Behenylalkohol | 1,00 |
| Cetylalkohol | 3,00 |
| Butylenglycol Dicaprylat/Dicaprat | 1,00 |
| Caprylsäure/Caprinsäure Triglycerid | 2,00 |
| Jojobaöl | 1,00 |
| Cyclomethicon | 3,00 |
| Dimethicon | 1,00 |
| Dicaprylylcarbonat | 3,00 |
| Ethylhexylcyanodiphenylacrylat | 5,00 |
| 2-Hydroxy-4-Methoxy- benzophenon | 3,00 |
| Phenylbenzimidazolsulfonsäure | 2,00 |
| Acetylsalicylsäure (Mikrokapseln gem. Herstellungsbeispiel Ia) | 0,20 |
| Trinatrium EDTA | 0,20 |
| Phenoxyethanol | 0,20 |
| p-Hydroxybenzoesäurealkylester (Paraben) | 0,30 |
| Diazolidinylharnstoff | 0,20 |
| Ammoniumpolyacryloyldimethyltaurate | 0,30 |
| Glycerin | 5,00 |
| Butylenglycol | 3,00 |
| Wasser | Ad 100,00 |

| **Rezepturbeispiel 5 (O/W**-**Crème)** | **Gew**.- **%** |
|---|---|
| Glycerylsterat, selbstemulgierend | 2,00 |
| PEG-40-Stearat | 1,00 |
| Myristylmyristat | 1,00 |
| Cetearylalkohol | 2,00 |
| Sheabutter | 2,00 |
| C₁₂₋₁₅ Alkylbenzoat | 3,00 |
| Caprylsäure/Caprinsäure Triglycerid | 2,00 |
| Ethylhexylkokosfettsäureester | 1,00 |
| Vaseline | 2,00 |
| Cyclomethicon | 5,00 |
| TiO₂ | 1,00 |
| Ethylhexylmethoxycinnamat | 3,00 |
| Ethylhexylcyanodiphenylacrylat | 3,00 |
| 2-Hydroxy-4-Methoxybenzophenon | 2,00 |
| Acetyl-L-Carnitin (Mikrokapseln gemäß Herstellungsbeispiel IV) | 0,50 |
| Tocopherylacetat | 0,30 |
| Ascorbylglucosid | 0,10 |
| Iminodisuccinat, Natriumsalz | 0,10 |
| Phenoxyethanol | 0,20 |
| p-Hydroxybenzoesäurealkylester (Paraben) | 0,30 |
| Diazolidinylharnstoff | 0,10 |
| Polyacrylsäure (Carbomer) | 0,10 |
| Aluminium Stärke Octenylsuccinat | 0,50 |
| Glycerin | 5,00 |
| Wasser | Ad 100,00 |

| **Rezepturbeispiel 6 (O/W**-**Crème)** | **Gew.%** |
|---|---|
| Glycerylsterat, selbstemulgierend | 2,50 |
| PEG-40-Stearat | 1,00 |
| Cetearylalkohol | 2,00 |
| Hydrierte Kokosfettglyceride | 1,00 |
| Sheabutter | 2,00 |
| C₁₂₋₁₅ Alkylbenzoat | 4,00 |
| Caprylsäure/Caprinsäure Triglycerid | 1,00 |
| Octyldodecanol | 1,00 |
| Vaseline | 1,00 |
| Octamethyltetrasiloxan (Cyclomethicon) | 4,00 |
| TiO₂ | 1,00 |
| Ethylhexylcyanodiphenyl-acrylat (Octocrylen) | 5,00 |
| Phenylbenzimidazol-sulfonsäure | 0,50 |
| Grünteeextrakt (Mikrokapseln gem. Herstellungsbeispiel Ib) | 0,20 |
| Ascorbinsäure | 0,20 |
| Iminodisuccinat | 0,20 |
| Phenoxyethanol | 0,50 |
| p-Hydroxybenzoesäurealkylester (Paraben) | 0,10 |
| Diazolidinylharnstoff | 0,20 |
| Xanthan Gummi | 0,10 |
| Glycerin | 8,00 |
| Wasser | Ad 100,00 |

| **Rezepturbeispiel 7 (O/W-Crème)** | **Gew.-%** |
|---|---|
| Polyglyceryl-3-Methylglucosedistearat | 3,00 |
| Sorbitanstearat | 1,00 |
| Behenylalkohol | 2,00 |
| Cetylalkohol | 1,00 |
| C₁₂₋₁₅ Alkylbenzoat | 2,00 |
| Caprylsäure/Caprinsäure Triglycerid | 1,00 |
| Hydriertes Polydecen | 1,00 |
| Octamethyltetrasiloxan (Cyclomethicon) | 3,00 |
| Dicaprylylcarbonat | 2,00 |
| Ethylhexylmethoxycinnamat | 5,00 |
| Butylmethoxydibenzoylmethan | 2,00 |
| Carnosin (Mikrokapseln gem. Herstellungsbeispiel IIb) | 0,20 |
| Tocopherylacetat | 0,30 |
| Iminodisuccinat | 0,20 |
| Phenoxyethanol | 0,40 |
| Diazolidinylharnstoff | 0,20 |
| Ethanol denaturiert | 5,00 |
| Xanthan Gummi | 0,20 |
| Ammoniumacryloyldimethyltaurat/Vinylpyrrolidoncopolymere | 0,30 |
| Glycerin | 5,00 |
| Wasser | Ad 100,00 |

| **Rezepturbeispiel 8 (O/W**-**Crème)** | **Gew.-%** |
|---|---|
| Polyethylenglycol(21)stearyl-ether (Steareth-21) | 2,00 |
| Polyethylenglycol(2)stearyl-ether (Steareth-2) | 1,00 |
| Cetearylalkohol | 2,00 |
| Sheabutter | 1,00 |
| C₁₂₋₁₅ Alkylbenzoat | 5,00 |
| Octyldodecanol | 1,00 |
| Mineralöl | 1,00 |
| Octamethyltetrasiloxan (Cyclomethicon) | 2,00 |
| Dicaprylylether | 2,00 |
| TiO₂ | 1,00 |
| Ethylhexylmethoxycinnamat | 4,00 |
| Ethylhexyltriazon | 1,00 |
| Grüntee Extrakt (Mikrokapseln gem.Herstellungsbeispiel Ib) | 0,10 |
| Biotin | 0,02 |
| Trinatrium EDTA | 0,20 |
| Phenoxyethanol | 0,50 |
| Hexamidindiisethionat | 0,10 |
| lodopropinylbutylcarbamat | 0,20 |
| Polyacrylsäure (Carbomer) | 0,10 |
| Ammoniumacryloyldimethyltaurate/Vinylpyrrolidoncopolymere | 0,30 |
| Glycerin | 7,00 |
| Wasser | Ad 100,00 |

| **Rezepturbeispiel 9 (O/W-Crème)** | **Gew.-%** |
|---|---|
| Cetearylglucosid | 2,00 |
| Myristylmyristat | 1,00 |
| Stearylalkohol | 4,00 |
| C₁₂₋₁₅ Alkylbenzoat | 2,00 |
| Caprylsäure/Caprinsäure Triglycerid | 3,00 |
| Hydriertes Polydecen | 1,00 |
| Dicaprylylcarbonat | 3,00 |
| Polydecen | 4,00 |
| Ethylhexylmethoxycinnamat | 3,00 |
| Ethylhexylcyanodiphenyl-acrylat (Octocrylen) | 3,00 |
| Butylmethoxydibenzoylmethan | 1,00 |
| Isoflavonoid-Extrakt (Mikrokapseln gem. Herstellungsbeispiel IIIa) | 0,10 |
| Ubichinon Q10 | 0,10 |
| Tocopherylacetat | 1,00 |
| Trinatrium EDTA | 0,10 |
| p-Hydroxybenzoesäurealkylester (Paraben) | 0,40 |
| Diazolidinylharnstoff | 0,10 |
| 2-Ethylhexylglycerinether (Octoxyglycerin) | 0,40 |
| Xanthan Gummi | 0,10 |
| Ammoniumpolyacryloyldimethyltaurate | 0,30 |
| Aluminium Stärke Octenylsuccinate | 0,50 |
| Glycerin | 3,00 |
| Butylenglycol | 3,00 |
| Wasser | Ad 100,00 |

| **Rezepturbeispiel 10 (O/W**-**Crème)** | **Gew.-%** |
|---|---|
| Glycerylsterat, selbstemulgierend | 1,00 |
| Stearinsäure | 2,50 |
| Behenylalkohol | 2,00 |
| Cetylalkohol | 3,00 |
| Hydrierte Kokosfettglyceride | 1,00 |
| C₁₂₋₁₅ Alkylbenzoat | 2,00 |
| Ethylhexylkokosfettsäureester | 2,00 |
| Octyldodecanol | 2,00 |
| Octamethyltetrasiloxan (Cyclomethicon) | 2,00 |
| Dimethylpolysiloxan (Dimethicon) | 1,00 |
| Dicaprylylcarbonat | 4,00 |
| TiO₂ | 1,00 |
| Ethylhexylmethoxycinnamat | 7,00 |
| Phenylbenzimidazol-sulfonsäure | 1,00 |
| 2-Hydroxy-4-Methoxy- benzophenon | 2,00 |
| Folsäure (Mikrokapseln gem. Herstellungsbeispiel IVa) | 0,20 |
| Tocopherylacetat | 0,50 |
| Lactoferrin | 0,05 |
| Iminodisuccinat | 0,10 |
| Phenoxyethanol | 0,30 |
| p-Hydroxybenzoesäurealkylester (Paraben) | 0,60 |
| Ethanol denaturiert | 3,00 |
| Polyacrylsäure (Carbomer) | 0,20 |
| Glycerin | 7,00 |
| Wasser | Ad 100,00 |

| **Rezepturbeispiel 11 (Hydrodispersion/Gelcreme)** | Gew.-% |
|---|---|
| Cetylalkohol | 2,00 |
| Sheabutter | 1,00 |
| Caprylsäure/Caprinsäure Triglycerid | 2,00 |
| Octyldodecanol | 1,00 |
| Octamethyltetrasiloxan (Cyclomethicon) | 5,00 |
| Dimethylpolysiloxan (Dimethicon) | 1,00 |
| Polydecen | 2,00 |
| Ethylhexylmethoxycinnamat | 3,00 |
| Bis-Ethylhexyloxyphenol-methoxyphenyltriazin | 0,50 |
| Acetyl-L-Carnitin (Mikrokapseln gem. Herstellungsbeispiel I) | 0,10 |
| Natriumascorbylphosphat | 0,05 |
| Iminodisuccinat | 0,20 |
| Phenoxyethanol | 0,30 |
| p-Hydroxybenzoesäurealkylester (Paraben) | 0,40 |
| Vernetztes Alkylacrylat (Alkylacrylate Cosspolymer) | 0,20 |
| Glycerin | 7,00 |
| Wasser | Ad 100,00 |

| **Rezepturbeispiel 12 (Foundation)** | **Gew.%** |
|---|---|
| Glycerylstearatcitrat | 3,50 |
| Cetylalkohol | 0,75 |
| Carbomer | 0,10 |
| Ammoniumacryloyldimethyltaurate/Vinylpyrrolidoncopolymere | 0,50 |
| Caprylsäure / Caprinsäure Triglycerid | 2,00 |
| Dicaprylylether | 2,00 |
| Cyclomethicon | 4,00 |
| PPG -15 Stearylether | 3,00 |
| Acetyliertes Lanolinöl | 0,20 |
| Ethylhexylmethoxycinnamat | 2,00 |
| TiO₂ | 2,00 |
| Bis-Ethylhexyloxyphenol-methoxyphenyltriazin | 1,00 |
| Niacinamid (Mikrokapseln gemäß Herstellungsbeispiel Ic) | 0,20 |
| Silikon | 0,80 |
| Eisenoxide | 2,40 |
| Titandioxid | 5,60 |
| ZnO | 1,00 |
| Polymethylsilsesquioxan (Tospearl 2000B) | 2,00 |
| EDTA | 0,60 |
| Glycerin | 5,00 |
| Phenoxyethanol und Paraben (Phenonip) | 0,50 |
| Imidazonidinylharnstoff | 0,30 |
| Wasser | ad 100,00 |

| **Rezepturbeispiel 13 (Foundation)** | **Gew.-%** |
|---|---|
| PEG-30-stearat | 1,50 |
| Gylcerylstearat | 0,50 |
| Ceteareth-20 | 1,00 |
| Stearinsäure | 2,00 |
| Cetylalkohol | 0,50 |
| Veegum K = Mg-Al-Silicate | 0,80 |
| Dimethicon | 3,00 |
| C₁₂₋₁₅ Alkyl Benzoate | 2,00 |
| Cetearyloctanoat | 2,00 |
| Squalan | 1,00 |
| Isopropylpalmitat | 1,00 |
| PPG -15 Stearylether | 2,00 |
| Hydrierte Kokos-Glyceride | 2,00 |
| Stearyldimethicon | 9,00 |
| Acetyliertes Lanolinöl | 2,00 |
| Ethylhexylmethoxycinnamat | 2,00 |
| Ethylhexyltriazon | 2,00 |
| Creatin (Mikrokapseln gem. Herstellungsbeispiel Ia) | 0,20 |
| Biotin | 0,02 |
| Lauroyllysin | 0,50 |
| Kaolin | 0,50 |
| Eisenoxide | 1,20 |
| Titandioxid | 3,80 |
| Interferenz Pigmente | 0,20 |
| Pigment Low Lustre | 0,20 |
| EDTA | 0,10 |
| Glycerin | 2,00 |
| Wasser | ad 100,00 |

| **Rezepturbeispiel 14 (Foundation)** | **Gew.-%** |
|---|---|
| Polyglyceryl-3 Methylglucose stearat | 3,50 |
| Sorbinsäurestearat | 1,50 |
| Cetylalkohol | 1,00 |
| Hydroxyethylcellulose | 0,10 |
| Caprylsäure / Caprinsäure Triglycerid | 3,00 |
| Dicaprylylether | 3,00 |
| Octyldodecanol | 3,00 |
| Dimethicon | 3,00 |
| Cyclomethicon | 3,00 |
| Bis-Ethylhexyloxyphenol-methoxyphenyltriazin | 2,00 |
| Pyridoxin (Mikrokapseln gem. Herstellungsbeispiel IVb) | 0,10 |
| Nylon-12 | 5,00 |
| Lauroyllysin | 0,50 |
| Kaolin | 1,00 |
| Eisenoxide | 2,60 |
| Titandioxid | 4,50 |
| EDTA | 1,00 |
| Glycerin | 5,00 |
| Wasser | ad 100 |

| **Rezepturbeispiel 15 (Foundation)** | **Gew.-%** |
|---|---|
| Gylcerylstearat | 2,00 |
| Stearinsäure | 1,80 |
| Lecithin | 0,50 |
| Veegum K = Mg-Al-Silicate | 1,00 |
| Xanthan Gum | 0,20 |
| Aluminium Stärke Octenylsuccinate | 1,00 |
| Caprylsäure / Caprinsäure Triglycerid | 2,00 |
| Dicaprylylether | 3,00 |
| Dimethicon | 3,00 |
| Cyclomethicon | 2,00 |
| Squalan | 6,00 |
| Isopropylpalmitat | 2,00 |
| Ethylhexylmethoxycinnamat | 2,00 |
| TiO₂ | 2,00 |
| Ethylhexyltriazon | 1,00 |
| Kreatin (Mikrokapseln gem. Herstellungsbeispiel Id) | 0,20 |
| Kaolin | 2,00 |
| Eisenoxide | 3,00 |
| Titandioxid | 6,50 |
| Interferenz Pigmente | 0,50 |
| Pigment Low Lustre | 0,20 |
| EDTA | 1,00 |
| Glycerin | 10,00 |
| Phenoxyethanol und Paraben (Phenonip) | 1,00 |
| Imidazonidinylharnstoff | 0,25 |
| Wasser | ad 100,00 |

## Patentansprüche

1. Wachsbeschichtete kosmetische und/oder dermatologische Wirkstoffpartikel erhältlich durch Behandeln der in Form einer Wirbelschicht vorgelegten unbeschichteten Wirkstoffpartikel durch Einbringen von geschmolzenem Wachs oder Wachsmischungen in die Wirbelschicht wobei das Wachs auf den Partikeln erstarrend abgeschieden wird.

2. Beschichtete Partikel nach Anspruch 1 **dadurch gekennzeichnet, dass** die mittlere Partikelgröße der zu beschichtenden Partikel 1 bis 200 µm, bevorzugt 5 bis 100 µm beträgt (gemessen durch statische Lichtstreuung im Mastersizer S der Firma Malvem mit einem Helium Neon Laser bei 632,8 nm in Wasser).

3. Beschichtete Partikel nach einem der vorangehenden Ansprüche **dadurch gekennzeichnet, dass** die Grenzen des Schmelzbereiches des Wachses oder der Wachsmischung zwischen 35 °C und 80° C liegen.

4. Beschichtete Partikel nach einem der vorangehenden Ansprüche **dadurch gekennzeichnet, dass** die mittlere Schichtdicke der Beschichtung 0,001 bis 50 µm, bevorzugt 0,1-10 µm, besonders bevorzugt 0,5 bis 5 µm beträgt (gemessen durch statischen Lichtstreuung im Mastersizer S der Firma Malvem mit einem Helium Neon Laser bei 632,8 nm in Wasser).

5. Beschichtete Partikel nach einem der vorangehenden Ansprüche, bei dem der Wirkstoff eine hydrophile Substanz darstellt.

6. Beschichtete Partikel nach einem der vorhergehenden Ansprüche **dadurch gekennzeichnet, dass** das Wachs aus der Gruppe der natürlichen Wachse, bevorzugt Bienenwachs, Carnaubauwachs, Candelillawachs, Shellackwachs, Kohlenwasserstoffe/ Paraffinwachse, bevorzugt Alkane, Isoalkane, Cycloalkane, Mono-, Di - und Triglyceride aus höheren gesättigten Fettsäuren mit 10 - 30 Kohlenstoffatomen, bevorzugt Glyceryltrilaureat, höhere gesättigte Fettalkohole, bevorzugt solche mit 14 - 30 Kohlenstoffatomen besonders bevorzugt, Cetostearylalkohol, synthetische Ester, bevorzugt Cetylpalmitat, Polymerwachse, bevorzugt Polyethylen, Polypropylen, Polyvinylether, Copolymere, bevorzugt solche aus Ethylen- und Vinylacetat, gewählt wird.

7. Beschichtete Partikel nach einem der vorhergehenden Ansprüche **dadurch gekennzeichnet, dass** der Wirkstoff aus der Gruppe der Enzyme, der Peptide, der Nucleotide, der pflanzlichen Polyphenole, Kreatin, Kreatinderivate, Ascorbinsäure, Carnitin und/oder Carnitinderivate, der Isoflavoncide, der B-Vitamine gewählt wird.

8. Kosmetische und/ oder dermatologische Zubereitungen enthaltend beschichtete Partikel nach einem der vorhergehenden Ansprüche.

9. Verwendung von kosmetischen und/oder dermatologischen Zubereitungen nach Anspruch 10 zur Pflege der Haut, der trockenen Haut, der Altershaut und der UV-geschädigten Haut.

## Claims

1. Wax-coated cosmetic and/or dermatological active ingredient particles obtainable by treating the uncoated active ingredient particles initially charged in the form of a fluidized bed by incorporating molten wax or wax mixtures into the fluidized bed, where the wax is deposited on the particles in a solidifying manner.

2. Coated particles according to Claim 1, **characterized in that** the average particle size of the particles to be coated is 1 to 200 µm, preferably 5 to 100 µm (measured by statistical light scattering in the Mastersizer S from Malvern with a helium neon laser at 632.8 nm in water).

3. Coated particles according to one of the preceding claims, **characterized in that** the limits of the melting range of the wax or the wax mixture are between 35°C and 80°C.

4. Coated particles according to one of the preceding claims, **characterized in that** the average layer thickness of the coating is 0.001 to 50 µm, preferably 0.1-10 µm, particularly preferably 0.5 to 5 µm (measured by statistical light scattering in the Mastersizer S from Malvern with a helium neon laser at 632.8 nm in water).

5. Coated particles according to one of the preceding claims, in which the active ingredient is a hydrophilic substance.

6. Coated particles according to one of the preceding claims, **characterized in that** the wax is selected from the group of natural waxes, preferably beeswax, carnauba wax, candelilla wax, shellac wax, hydrocarbons/paraffin waxes, preferably alkanes, isoalkanes, cycloalkanes, mono-, di- and triglycerides of higher saturated fatty acids with 10-30 carbon atoms, preferably glyceryl trilaureate, higher saturated fatty alcohols, preferably those with 14-30 carbon atoms, particularly preferably cetostearyl alcohol, synthetic esters, preferably cetyl palmitate, polymer waxes, preferably polyethylene, polypropylene, polyvinyl ether, copolymers, preferably those of ethylene acetate and vinyl acetate.

7. Coated particles according to one of the preceding claims, **characterized in that** the active ingredient is selected from the group of the enzymes, the peptides, the nucleotides, the vegetable polyphenols, creatine, creatine derivatives, ascorbic acid, carnitine and/or carnitine derivatives, the isoflavoncids, and the B vitamins.

8. Cosmetic and/or dermatological preparations comprising coated particles according to one of the preceding claims.

9. Use of cosmetic and/or dermatological preparations according to Claim 10 for caring for the skin, dry skin, ageing skin and UV-damaged skin.

## Revendications

1. Particules cosmétiques et/ou dermatologiques de substance active, recouvertes par une cire, obtenues par traitement des particules de substance active non revêtues disposées au préalable dans un lit fluidisé par introduction de cire fondue ou de mélanges de cires dans le lit fluidisé, la cire étant déposée sur les particules tout en se solidifiant.

2. Particules revêtues selon la revendication 1, **caractérisées en ce que** la grosseur moyenne des particules à revêtir est de 1 à 200 µm, de préférence de 5 à 100 µm (mesurée par dispersion statique de la lumière dans un Mastersizer S de la société Malvern à l'aide d'un laser à hélium-néon à 632,8 nm dans l'eau).

3. Particules revêtues selon l'une quelconque des revendications précédentes, **caractérisées en ce que** les limites de la plage de fusion de la cire ou du mélange de cires sont situées entre 35°C et 80°C.

4. Particules revêtues selon l'une quelconque des revendications précédentes, **caractérisées en ce que** l'épaisseur moyenne de couche du revêtement est de 0,001 à 50 µm, de préférence de 0,1-10 µm de manière particulièrement préférée de 0,5 à 5 µm (mesurée par dispersion statique de la lumière dans un Mastersizer S de la société Malvern à l'aide d'un laser à hélium-néon à 632,8 nm dans l'eau).

5. Particules revêtues selon l'une quelconque des revendications précédentes, dans lesquelles la substance active est une substance hydrophile.

6. Particules revêtues selon l'une quelconque des revendications précédentes, **caractérisées en ce que** la cire est choisie dans le groupe formé par les cires naturelles, de préférence la cire d'abeille, la cire de carnauba, la cire de candelilla, la cire de shellac, les cires hydrocarbonées/paraffiniques, de préférence les alcanes, les isoalcanes, les cycloalcanes, les monoglycérides, les diglycérides et les triglycérides d'acides gras saturés supérieurs comprenant 10-30 atomes de carbone, de préférence le trilauréate de glycéryle, les alcools gras saturés supérieurs, de préférence ceux comprenant 14-30 atomes de carbone, de manière particulièrement préférée l'alcool cétostéarylique, les esters synthétiques, de préférence le palmitate de cétyle, les cires polymères, de préférence le polyéthylène, le polypropylène, le polyvinyléther, les copolymères, de préférence ceux d'éthylène et d'acétate de vinyle.

7. Particules revêtues selon l'une quelconque des revendications précédentes, **caractérisées en ce que** la substance active est choisie dans le groupe formé par les enzymes, les peptides, les nucléotides, les polyphénols végétaux, la créatine, les dérivés de créatine, l'acide ascorbique, la carnitine et/ou les dérivés de carnitine, les isoflavonoïdes, la vitamine B.

8. Préparations cosmétiques et/ou dermatologiques contenant des particules revêtues selon l'une quelconque des revendications précédentes.

9. Utilisation de préparations cosmétiques et/ou dermatologiques selon la revendication 10 pour les soins de la peau, de la peau sèche, de la peau mature et de la peau abîmée par les UV.
